# EUROPÄISCHE PATENTSCHRIFT

(11) **EP 0 647 564 B1**
(45) Veröffentlichungstag und Bekanntmachung des Hinweises auf die Patenterteilung: **12.02.1997**
(21) Anmeldenummer: 93116431.3
(22) Anmeldetag: 11.10.1993
(51) Int. Cl.: B65B 55/10

(54) **Verfahren und Maschine zur aseptischen Abfüllung von Nahrungs- oder Genussmitteln**
Process and machine for aseptic filling of food products
Procédé et machine pour le remplissage aseptique de denrées alimentaires

(43) Veröffentlichungstag der Anmeldung: 12.04.1995
(73) Patentinhaber: GEA Finnah GmbH, D-48683 Ahaus (DE)
(72) Erfinder: Finnah, Josef, D-48663 Ahaus (DE)
(74) Vertreter: Busse & Busse Patentanwälte

(56) Entgegenhaltungen:
- EP-A- 0 431 392
- DE-A- 3 016 266
- VERPACKUNGS-RUNDSCHAU Bd. 33, Nr. 8 , 1982 , HEUSENSTAMM (DE) Seiten 47 - 50 GERHARD CERNY 'Entkeimen von Packstoffoberflächen mittels Heissdampf(..)'

## Beschreibung

Die Erfindung bezieht sich auf ein Verfahren und eine Maschine zur aseptischen Abfüllung von Nahrungs- oder Genußmitteln gemäß dem Oberbegriff der Ansprüche 1 oder 2 bzw. 6.

Bei einem bekannten Verfahren (Verpackungsrundschau 33 (1982) Nr. 8, Seiten 47-50; EP 0 045 389 B1) wird nach der Becherformung durch Tiefziehen die Becherfolie mit Dampf beaufschlagt, der die Sterilisation der Becherfolie auf deren Befüllungsseite herbeiführt. Bei diesem Vorgang wird die Becherfolie rückseitig durch die Wandung des Formwerkzeugs gekühlt. Die Deckelfolie aus Aluminium wird bei Passieren einer Dampfkammer beidseitig sterilisiert. Die auf diese Weise erfolgte Sterilisation der Becherfolie läßt zu wünschen übrig und schließt Gefahren der Reinfektion nicht aus. Bei aus Kunststoff bestehenden Deckelfolien führt eine gleichzeitige beidseitige Dampfbeaufschlagung zu Verzugserscheinungen. Ferner ist es bekannt, zu Sterilisationszwecken die Becherfolie vor ihrer Verformung durch ein Bad mit Wasserstoffperoxid hindurchzuführen, das eine beidseitige Foliensterilisation bewirkt (DE 30 28 208 A1).

Der Erfindung liegt die Aufgabe zugrunde, ein Verfahren und eine Maschine zur aseptischen Abfüllung von Nahrungs- oder Genußmitteln in Kunststoffbecher zu schaffen, die eine schonende und sichere Sterilisation des Gutaufnahmeraumes der Becher erbringen und in Abfüllanlagen einsetzbar sind, die mit hoher Taktgeschwindigkeit arbeiten.

Die Erfindung löst diese Aufgabe durch ein Verfahren mit den Merkmalen des Anspruchs 1 sowie eine Maschine mit den Merkmalen des Anspruchs 6. Hinsichtlich wesentlicher weiterer Ausgestaltungen wird auf die Ansprüche 2 bis 5 bzw. 7 bis 14 verwiesen.

Verfahren und Maschine nach der Erfindung ermöglichen auf einfache und kostengünstige Weise die Ausnutzung der sterilisierenden Wirkung von Wasserdampf auf beiden Seiten von aus Kunststoff bestehenden Folien über eine hinreichende Einwirkzeit, da die Folien mit geringem Aufwand über einen Zeitraum von mehreren Arbeitstakten einer Abfüllanlage und damit lange genug behandelbar sind, um eine sichere Keimabtötung zu erreichen. Die beidseitige Sterilisierung vermeidet ein Einschleppen von Keimen in den Abfüllbereich und gewährleistet damit eine zuverlässige aseptische Abfüllung von Produkten in die Becher.

Mit der wechselseitigen, in zwei aufeinanderfolgenden Vorgängen auf flache Längenabschnitte der Folien einwirkenden Dampf- und Kühlbeaufschlagung kann mit relativ hohen Dampftemperaturen die Folienfläche beaufschlagt und eine vollständige Flächensterilisation erreicht werden, ohne daß auch bei längerer Einwirkdauer des Dampfes die Formstabilität der Folien leidet.

Die Foliensterilisation in einem von weiteren Bearbeitungsvorgängen getrennten Vorgang eröffnet die Möglichkeit, mit unterschiedlichen Vorschubgeschwindigkeiten zu arbeiten, so daß kurze Taktzeiten in den Hauptstationen und dennoch eine für die Sterilisation hinreichende Einwirkdauer des Dampfes auf der jeweiligen Folienseite erreichbar sind.

Hinsichtlich weiterer Vorteile und Einzelheiten der Erfindung wird auf die nachfolgende Beschreibung und die Zeichnung verwiesen, in der ein Ausführungsbeispiel des Gegenstandes der Erfindung schematisch näher veranschaulicht ist. In der Zeichnung zeigen:
- Fig. 1: eine Prinzipdarstellung einer Verpackungsmaschine zur aseptischen Abfüllung gemäß der Erfindung,
- Fig. 2: eine vergrößerte Ausschnittsdarstellung einer Sterilisationsvorrichtung für die Becherfolie mit einem anschließenden Steriltunnel gemäß Ausschnitt 5 in Fig. 1,
- Fig. 3: eine vergrößerte Prinzipdarstellung einer dem Steriltunnel zugeordneten Deckelfolien-Sterilisationsvorrichtung gemäß Ausschnitt 7 in Fig. 1,
- Fig. 4: eine vergrößerte Einzeldarstellung einer Sterilisationsvorrichtung für die Deckelfolie ähnlich Fig. 3,
- Fig. 5: eine vergrößerte Teil-Darstellung des Ausschnitts 5 in Fig. 1 im Bereich des Zulaufes der Becherfolie in die Sterilisationsvorrichtung,
- Fig. 6: eine teilweise geschnittene Darstellung des Einlaufes in die Sterilisationsvorrichtung nach der Schnittlinie VI-VI in Fig. 5,
- Fig. 7: eine Darstellung eines Bereichs des Steriltunnels nach der Linie VII-VII in Fig. 2,
- Fig. 8: eine vergrößerte Darstellung eines Bereichs der Becherformung nach der Linie VIII-VIII in Fig. 2,
- Fig. 9: eine vergrößerte Darstellung eines Bereichs der Deckelansiegelung nach der Linie IX-IX in Fig. 3 und
- Fig. 10: eine Darstellung eines Bereichs der Voransiegelung nach der Linie X-X in Fig. 3.

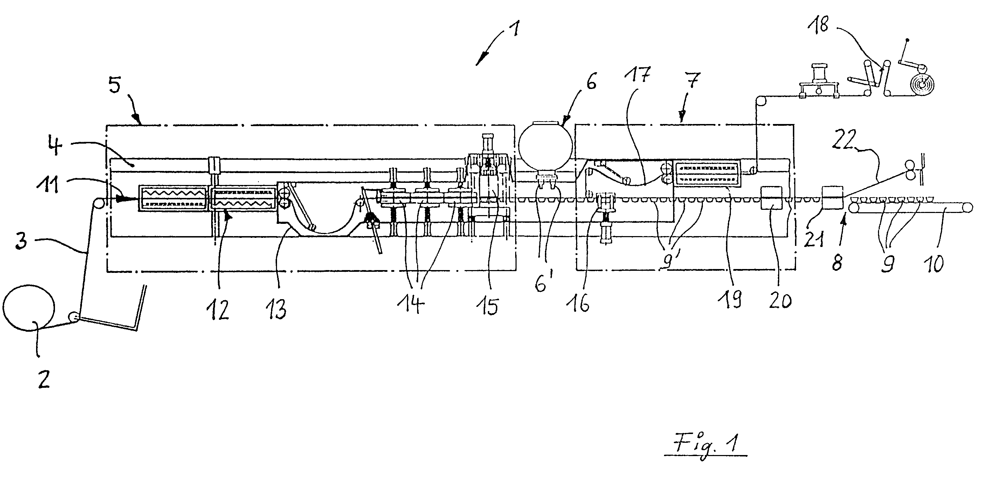

In Fig. 1 ist eine insgesamt mit 1 bezeichnete Abfüllmaschine dargestellt, die mit einer von einer Vorratsrolle 2 abrollenden Becherfolie 3 beschickt wird. Die Becherfolie 3 bzw. Folienbahn passiert innerhalb eines Maschinenrahmens 4 der Abfüllmaschine 1 einen vorderen Bereich 5, eine Abfüllvorrichtung 6 und danach einen hinteren Bereich 7, und in einem Endbereich 8 gehen gefüllte einzelne Kunststoffbecher 9 auf ein Transportband 10 über.

In der mit einem Pfeil 11 angedeuteten Vorschubrichtung der Becherfolie 3 sind im vorderen Bereich 5 hintereinander eine Sterilisationsvorrichtung 12, die unmittelbar in einen sich bis in den hinteren Bereich 7 erstreckenden Steriltunnel 13 mündet, drei Kontaktheizplatten 14 und eine Becherformstation 15 angeordnet. Daran schließt sich hinter der mit zumindest ihren Füllorganen 6' in den Steriltunnel 13 hineinragenden Abfüllvorrichtung 6 im hinteren Bereich 7 der Abfüllmaschine 1 eine Voransiegelvorrichtung 16 an, in deren Arbeitsbereich im Steriltunnel 13 eine Deckelfolie 17 von einer Deckelfolienvorratsrolle 18 über eine Sterilisationsvorrichtung 19 zugeführt wird. Die in der Voransiegelungsvorrichtung 16 entlang ihrer Außenränder mit den Außenrändern der Deckelfolie 17 verbundene Becherfolie 3 verläßt mit gefüllten Becherteilen 9' in Vorschubrichtung 11 den Steriltunnel 13 und wird mit der Deckelfolie 17 durch eine Versiegelungsvorrichtung 20 voll versiegelt, so daß danach im Endbereich 8 mittels einer Stanze 21 die über das Transportband 10 weiterbeförderten Kunststoffbecher 9 vereinzelt und von der dabei als Abfall entstehenden Stanzgitterbahn 22 getrennt werden können.

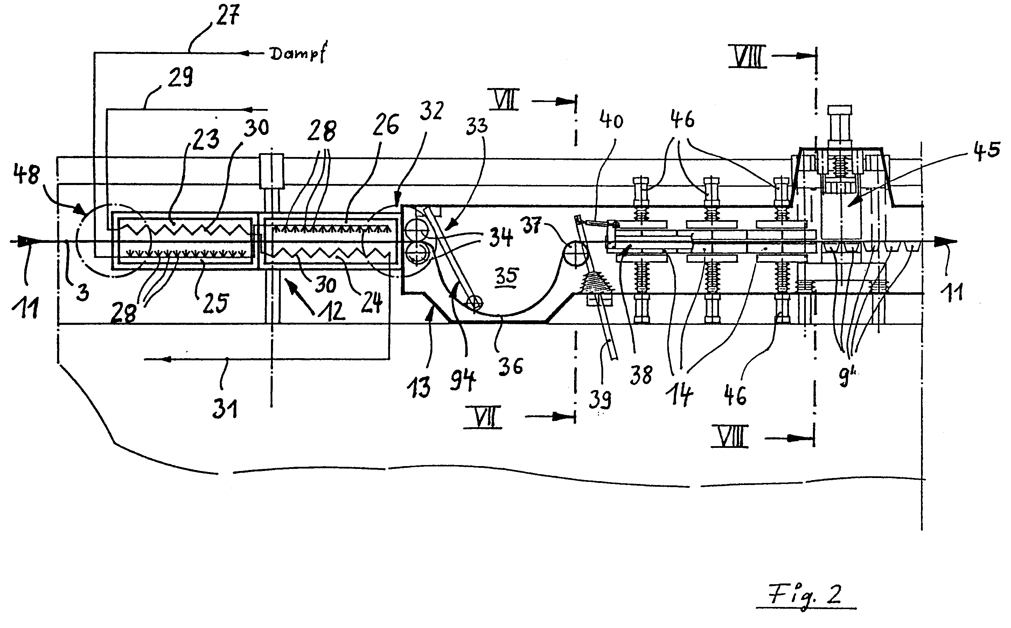

In Fig. 2 ist der vordere Bereich 5 der Abfüllmaschine 1 als vergrößerter Ausschnitt dargestellt, wobei die vorteilhafte Ausbildung der Sterilisationsvorrichtung 12 mit zwei hintereinander angeordneten Gruppen von plattenförmigen Werkzeugteilen deutlich wird, die jeweils einen Längenabschnitt der flachen Becherfolie 3 zwischen sich aufnehmen können. Dabei ist jeweils ein Werkzeugteil jeder Gruppe als Kontaktkühlplatte 23 bzw. 24 und der gegenüberliegende Werkzeugteil als Dampfverteilerplatte 25 bzw. 26 ausgebildet. Die Werkzeugteile, bevorzugt nur die jeweils unteren Werkzeugteile 24,25, sind mittels eines Hubantriebes (nicht dargestellt) aus ihrer dargestellten Betriebsstellung in eine den Vorschub der Becherfolie 3 in Pfeilrichtung 11 ermöglichende Offenstellung verbringbar. Sie können mittels einer Verriegelungsvorrichtung in ihrer Betriebsstellung (Fig. 2) aneinander festgelegt werden.

Die Sterilisationsvorrichtung 12 wird dabei über eine Zuleitung 27 aus einem Dampferzeuger (nicht dargestellt) derart mit Dampf versorgt, daß im Bereich der jeweiligen Dampfverteilerplatten 25 bzw. 26 über eine der Becherfolie 3 zugewandte Fläche verteilt angeordnete Anzahl von Austrittsöffnungen 28 eine hinreichende Menge Dampf zugeführt und über die gesamte gegenüberliegende Becherfolienfläche verteilt werden kann. Die Austrittsöffnungen 28 können dabei vorteilhaft als Düsen ausgebildet sein, mit denen der Dampf auf die jeweilige Seite der flachen Becherfolie 3 gerichtet wird. In diesem flachen Zustand erfährt die Becherfolie 3 erst auf einem Abschnitt auf ihrer Unterseite und dann im zweiten Behandlungsvorgang im gleichen Abschnitt auf ihrer Oberseite eine vollständige Sterilisation, bei der keine Unebenheiten oder Formausnehmungen eine gleichmäßige Dampfeinwirkung beeinträchtigen.

Über eine weitere Zuleitung 29 wird gleichzeitig mit der Dampfbeaufschlagung der Dampfverteilerplatten 25,26 ein entsprechendes Kühlmedium den Kontaktkühlplatten 23,24 zugeleitet und nach dem Passieren von Kühlschleifen 30 über eine Rückführungsleitung 31 abgeführt, so daß das Kühlmedium über einen Wärmetauscher (nicht dargestellt) im Kreislauf umgewälzt werden kann.

Das Kühlen der einen Seite der Becherfolie 3 während der gleichzeitigen Dampf-Sterilisation der Gegenseite vermeidet Beeinträchtigungen des Folienmaterials durch Überhitzung auch bei höheren, die Erweichungstemperatur erheblich übersteigenden Dampftemperaturen, z.B. von 120 - 145°C, und längeren Einwirkzeiten, z.B. von 8 Sekunden oder mehr. Dies gilt auch für unterschiedlich dünne Folien.

Die Sterilisationsvorrichtung 12 bildet mit ihren beiden Werkzeuggruppen 23,25 bzw. 24,26 eine insgesamt gekapselte Einheit, die unmittelbar dem mit Sterilluft beschickten Steriltunnel 13 der Abfüllmaschine 1 vorgeordnet ist, wobei in einem Bereich 32 ein von der Umgebung abgeschirmter Übergang der Becherfolie 3 in den Steriltunnel 13 erfolgt. Damit ist sichergestellt, daß die in zwei aufeinanderfolgenden Behandlungsschritten sterilisierte Becherfolie 3 bereits vor ihrer Plastifizierung und Umformung zu Bechern keimfrei und eine Einschleppung von Keimen in den Steriltunnel 13 vermieden ist, in dem durch die in den Steriltunnel 13 eingeblasene Sterilluft ein durchgehend keimfreier Innenraum ausgebildet ist.

Zur Bewegung der Becherfolie 3 durch die Sterilisationsvorrichtung 12 ist im Eingangsbereich des Steriltunnels 13 eine gesonderte Vorschubvorrichtung 33 vorgesehen, die separat angetriebene Vorschubwalzen 34 aufweist. Diese erteilen der Becherfolie 3 eine von der Vorschubbewegung der Becherfolie 3 in den nachfolgenden Bereichen der Abfüllmaschine 1 unabhängige Bewegung. Bevorzugt erteilt die Vorschubvorrichtung 33 in zeitlichen Abständen von mehreren, bevorzugt vier Arbeitstakten der Abfüllmaschine 1 (d.h. bei einer Arbeitstaktzeit von z.B. 2 Sekunden in einem zeitlichen Abstand von z.B. 8 Sekunden) einen Vorschub, der dem Vorschub der Becherfolie 3 im anschließenden Teil der Abfüllmaschine 1 zumindest innerhalb des gleichen zeitlichen Abstandes im wesentlichen gleich ist. Bei einem Vorschub im zeitlichen Abstand von vier Arbeitstakten beträgt dementsprechend der Vorschub durch die Vorschubwalzen 34 das Vierfache des Vorschubs der Becherfolie 3 während eines Arbeitstaktes der Abfüllmaschine 1 in dem Bereich hinter der Sterilisationsvorrichtung 12.

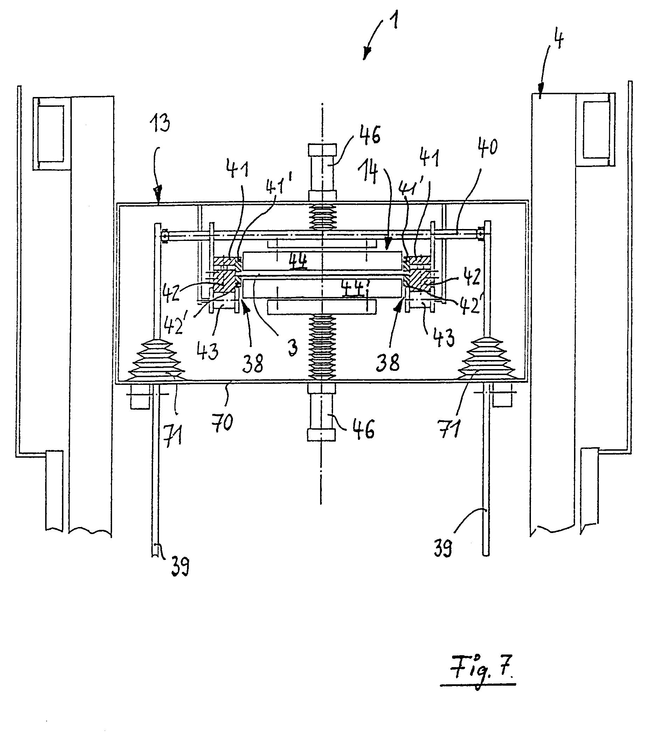

Dazu ist der Steriltunnel 13 hinter den Vorschubwalzen 34 mit einem erweiterten Aufnahmeraum 35 versehen, der eine etwa einem Vorschub der Vorrichtung 33 entsprechende Vorratsschlaufe 36 der Becherfolie 3 aufnehmen kann. Die Becherfolie 3 gelangt aus diesem Bereich der Vorratsschlaufe 36 über eine Umlenkrolle 37 in den Bereich der Kontaktheizplatten 14 und wird von da an durch zwei von einem Hauptantrieb (nicht dargestellt) der Abfüllmaschine 1 her angetriebene Vorschubbalken 38 (Fig. 7) taktweise weiterbewegt, die die Hauptvorschubvorrichtung der Abfüllmaschine 1 bilden.

Die beiden Vorschubbalken 38 greifen jeweils an den beiden Längsrändern der Becherfolie 3 an. Jeder Vorschubbalken 38 ist dazu mit einer Querstange 40 einer Antriebshebel 39 umfassenden Antriebsschwinge verbunden und umfaßt Hubantriebe zwischen einem Oberteil 41 und einem Unterteil 42, die mit Klemmleisten 41' und 42' die Becherfolie 3 randseitig erfassen und für eine Vorschubbewegung in Pfeilrichtung 11 mitnehmen können. Die Vorschubbalken 38 sind auf beiseitigen Rollführungen 43 vor- und zurückbeweglich abgestützt.

Im Bereich der drei Kontaktheizplatten 14 wird die Becherfolie 3 stufenweise plastifiziert, wobei nach Abschluß einer Vorschubbewegung für einen Arbeitstakt der Abfüllmaschine 1 bewegliche Oberplatten 44 und Unterplatten 44' (Fig. 7) über jeweils zugeordnete Druckluftzylinder 46 gegen die Becherfolie 3 eingerückt und vor Beginn einer nachfolgenden Vorschubbewegung wieder außer Eingriff bewegt werden. In der Phase der Plastifizierung der Becherfolie 3 mittels der Kontaktheizplatten 14 können im Bereich der Oberfläche der Becherfolie 3 Temperaturen erreicht werden, die ihrerseits einen Sterilisierungseffekt erbringen.

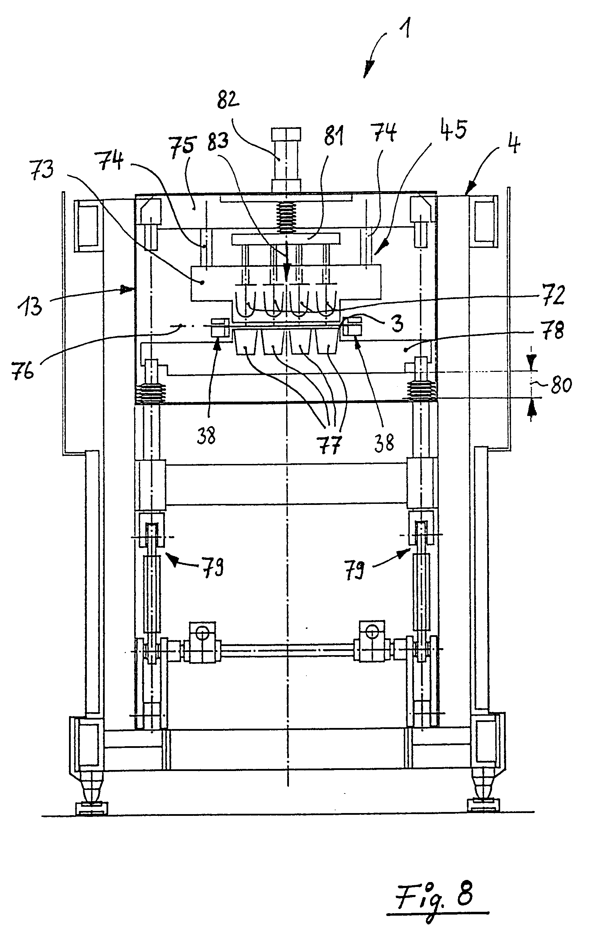

In der Becherformstation 15 werden die Becherteile 9' unter Wirkung eines entsprechenden Umformwerkzeuges 45 (Fig. 8) aus der plastifizierten Becherfolie 3 geformt und danach in den Bereich der Abfüllvorrichtung 6 vorgeschoben.

Durch die in Fig. 1 und Fig. 2 veranschaulichte Sterilisationsvorrichtung 12 kann die Becherfolie 3 im jeweiligen Dampfbeaufschlagungsbereich oberflächig auf ca. 120 - 145°C erwärmt und durch die gleichzeitige Kühlung der gegenüberliegenden Oberfläche der Becherfolie 3 über die jeweiligen Kontaktkühlplatten 23,24 soviel Wärme entzogen werden, daß die Temperatur an der gekühlten Oberfläche der Becherfolie 3 etwa 60°C nicht überschreitet, so daß thermische Verformungen der Becherfolie 3 ausgeschlossen sind. Die Haltezeit im Bereich der jeweiligen Dampfbeaufschlagung von vorzugsweise etwa ca. 5 bis 10 Sekunden erfordert lediglich eine entsprechende Bemessung der Länge der Sterilisationsvorrichtung 12 in Vorschubrichtung 11, die mit geringem Aufwand verwirklicht werden kann.

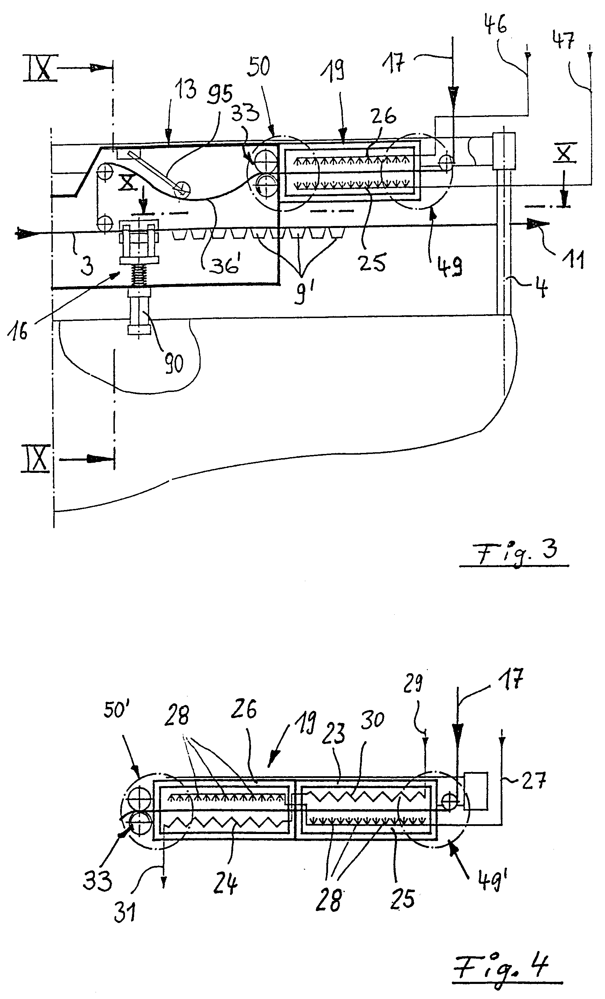

Der in Vergrößerung in Fig. 3 dargestellte hintere Bereich 7 der Abfüllmaschine 1 verdeutlicht die Sterilisationsvorrichtung 19 im Zuführungsbereich der Deckelfolie 17. Die Sterilisationsvorrichtung 19 hat eine Ausbildung, wie sie für eine Deckelfolie 17 aus Aluminium geeignet ist, die wegen der Hitzebeständigkeit des Materials eine gleichzeitige beidseitige Beaufschlagung mit Dampf erlaubt. Der Dampf wird über entsprechende Zuleitungen 46 und 47 zwei einander gegenüberliegenden Dampfverteilerplatten 25,26 zugeführt. Die vor dem Eintritt in den Steriltunnel 13 erfolgende Sterilisierung ist in diesem Falle ohne Kühlungsmaßnahmen durchführbar. Auch hier sichert die unmittelbare Anordnung der Sterilisationsvorrichtung 19 vor dem rückwärtigen Ende des Steriltunnels 13 einen zuverlässig keimfreien Einlauf der Deckelfolie 17 in den Steriltunnel 13 und in diesem zur Deckelvoransiegelungvorrichtung 16.

Für Deckelfolien 17 aus einem Kunststoffmaterial findet eine Sterilisationsvorrichtung 19' in einer Ausbildung Anwendung, die jener der Sterilisationsvorrichtung 12 für die Becherfolie 3 entspricht. Eine solche Ausführung zeigt Fig. 4, in der mit der Sterilisationsvorrichtung 12 in Fig. 2 baugleiche Teile mit gleichen Bezugszeichen versehen sind. Hinsichtlich Ausbildung und Funktion der Sterilisationsvorrichtung 19' wird auf die Ausführungen zur Sterilisationsvorrichtung 12 verwiesen. Auch für den Deckelfolienantrieb, die Schlaufenbildung etc. gilt das zu Fig. 2 ausgeführte sinngemäß.

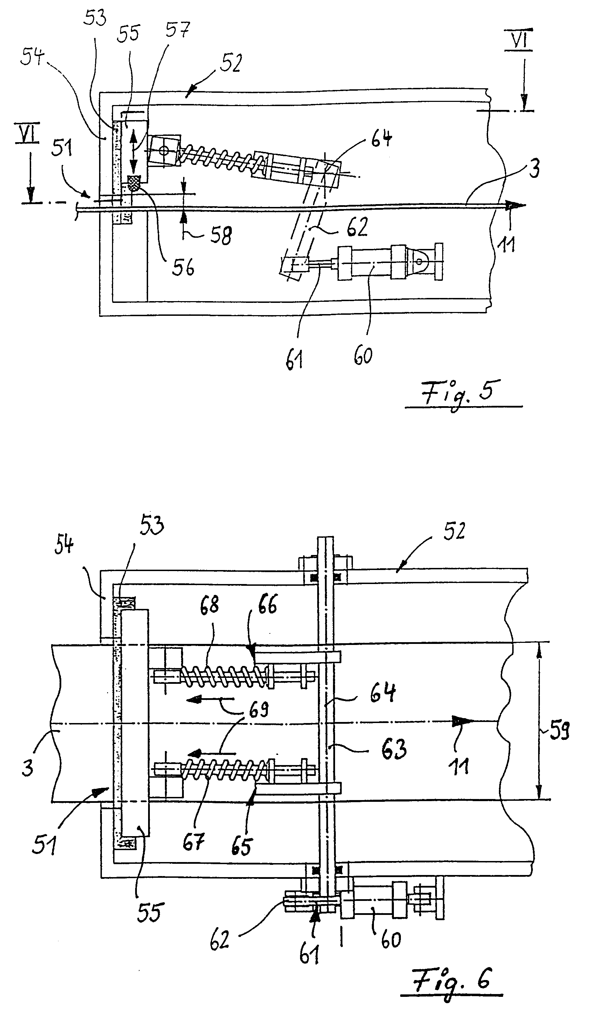

Für eine zuverlässige Sterilhaltung der Becherfolie 3 und der Deckelfolie 17 im Steriltunnel 13 kann eine den Eintritt keimhaltiger Umgebungsluft unterbindende Abdichtung erforderlich sein. Dies gilt vornehmlich für die Bereiche 48 (Fig. 2) und 49 bzw. 49' (Fig. 3,4). An den Übergängen in den Bereichen 32 (Fig. 2) und 50,50' (Fig. 3,4) können ebenfalls Abdichtungen vorgesehen sein. In Fig. 5 und 6 ist eine für die Bereiche 48,49 beispielhafte Gestaltung der Abdichtung einer Zuführöffnung 51 für die Becherfolie 3 bzw. Deckelfolie 17 zur Sterilisationsvorrichtung 12 bzw. 19,19' dargestellt, wie sie prinzipiell auch an Übergängen in den Bereichen 32,50,50' vorgesehen werden kann.

Fig. 5 und 6 verdeutlichen in zwei Ansichten ein Gehäuse 52, das die Sterilisationsvorrichtung 12 bzw. 19 oder 19' insgesamt umgibt. Im Bereich der Zuführöffnung 51 ist dabei eine Führungsleiste 53 angeordnet, die sich innenseitig an der Wand 54 des Gehäuses 52 abstützt und mit einer Durchgangsöffnung die Zuführöffnung 51 fortsetzt. An der Führungsleiste 53 ist ein Dichtbalken 55 geführt, der bei einem Andrücken an die Führungsleiste 53 an dieser in Dichtungseingriff gelangt. Eine Dichtungsleiste 56, insbesondere aus temperaturfestem Silikongummi, legt sich bei Abwärtsbewegung des Dichtbalkens 55 in Pfeilrichtung 57 derart auf die Becherfolie 3 auf, daß die Zuführöffnung 51 in voller Öffnungshöhe 58 verschlossen ist.

Der randseitig über die Becherfolienbreite 59 im Bereich der Zuführöffnung 51 überstehende Dichtbalken 55 ist mittels eines Hubzylinders 60, z.B. eines Druckluftzylinders, bewegbar, wobei die Hubbewegung der Kolbenstange 61 über einen gelenkig mit dieser verbundenen Hebel 62 auf eine mit diesem fest verbundene, das Gehäuse 52 durchgreifende Querwelle 63 übertragen wird, deren Schwenkung um die Mittelachse 64 auf Druckstangen 65,66 übertragen wird, die eine den Pfeilrichtungen 57 (Fig. 5) entsprechende Auf- und Abbewegung des Dichtbalkens 55 herbeiführen.

Den Druckstangen 65,66 ist jeweils eine Druckfeder 67,68 zugeordnet, welche die Druckstangen und damit den Dichtbalken 55 in einer Pfeilrichtung 69 an die Führungsleiste 53 andrücken.

Wie Fig. 7 zeigt, sind die zur Betätigung des Vorschubbalkens 38 vorgesehenen, den Boden 70 des Steriltunnels 13 durchgreifenden Antriebshebel 39 ebenso wie die in den Steriltunnel 13 eingreifenden Kolbenstangen der Antriebszylinder 46 für die Heizplatten 44,44' durch je ein Faltenbalg 71 mit einer zuverlässigen Abdichtung versehen.

Fig. 8 veranschaulicht beispielhaft ein Umformwerkzeug 45 mit vier Becherformwerkzeugen 72, die in einem Oberwerkzeug 73 geführt sind, welches ortsfest über Streben 74 gegen ein Joch 75 im Steriltunnel 13 abgestützt ist. Unterhalb einer Vorschubebene 76, in der sich die Becherfolie 3 bewegt, befinden sich den jeweiligen Becherformwerkzeugen 72 zugeordnete Becherformnester 77 in einem Unterwerkzeug 78. Das Unterwerkzeug 78 ist gegenüber dem ortsfesten Oberwerkzeug 73 über ein jeweils randseitig angreifendes Antriebshebelsystem 79 um einen Hubweg 80 soweit aus der Betriebsebene absenkbar, daß bei Bewegung der Becherfolie 3 in Pfeilrichtung 11 (Fig. 2) die ausgeformten Becherteile 9' frei zur Abfüllvorrichtung 6 hin vorgeschoben werden können (Fig. 2). Die dabei gleichzeitig in den Bereich zwischen die Becherformwerkzeuge 72,77 vorgeschobene, von den Kontaktheizplatten 14 plastifizierte, noch flache Becherfolie 3 kann nun zur Becherbildung in einem erneuten Arbeitsgang verformt werden.

Für eine Formung der Becher werden die Becherformwerkzeuge 72 über eine gemeinsame Trägerplatte 81 mittels eines obenliegenden Antriebszylinders 82 in Umformrichtung 83 auf die Becherfolie 3 und in die von dieser überdeckten Formnester 77 absenkt. Nach dieser mechanischen Vorformung erfolgt die Fertigformung der Becherteile 9' durch sterile Druckluft.

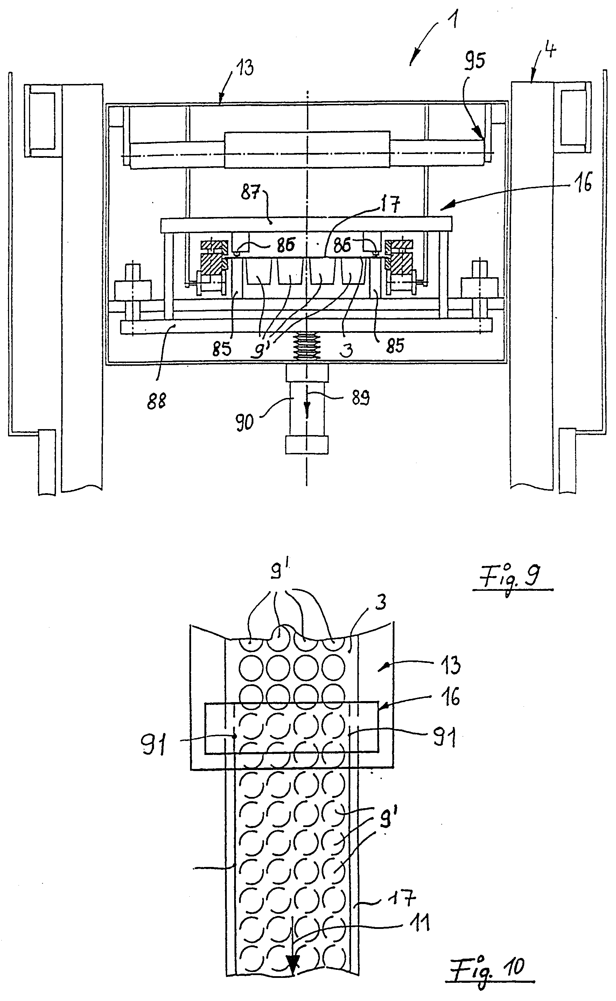

Die Becherfolie 3 mit ihren ausgeformten Becherteilen 9' wird in den Bereich der Abfüllvorrichtung 6 (Fig. 1) vorgeschoben, wo die Becherausnehmungen befüllt werden. Von dort gelangen die befüllten Becherteile 9' in den Bereich der Deckelvoransiegelvorrichtung 16, die in Fig. 9 in einem vergrößerten Querschnitt dargestellt ist. In der Station 16 liegt die Becherfolie 3 randseitig auf Stützkörpern 85 auf, denen jeweils ein Siegelglied 86 gegenüberliegend zugeordnet ist. Die Siegelglieder 86 sind dabei über ein Brückenteil 87 mit einem Hubbalken 88 verbunden, bei dessen Bewegung mittels eines Antriebszylinders 90 in Pfeilrichtung 89 die Siegelglieder 86 auf die Oberseite der inzwischen zugeführten, die Becherfolie 3 übergreifenden Deckelfolie 17 randeitig abgesenkt werden und die Deckelfolie 17 mit der Becherfolie 3 randseitig während des Vorschubtaktes verbunden wird.

Fig. 10 veranschaulicht in schematischer Draufsicht auf den Bereich der Voransiegelung, daß die Deckelfolie 17 vor dem Passieren des Endes des Steriltunnels 13 randseitig über eine siegelnaht 91 mit der Becherfolie 3 derart verbunden ist, daß beim Austritt in die Umgebung ein steriler Abschluß für das in den Bechern 9' befindliche Nahrungs- und Genußmittel erhalten bleibt. Hinter der Versiegelungsstation 20 (Fig. 1) ist dann eine beliebige Weiterverarbeitung zu Einzelbechern 9 oder Bechergruppen (nicht dargestellt) möglich.

Den Vorratsschlaufen 36 für die Becherfolie 3 bzw. 36' für die Deckelfolie 17 kann ein Taster zugeordnet sein, mittels dem die Länge der Schlaufe erfaßt und gegebenenfalls korrigiert werden kann. Dazu kann beispielsweise ein als Schaltschwinge ausgebildeter Taster 94 bzw. 95 (Fig. 2 und Fig. 3) vorgesehen sein, der bei Erreichen vorgegebener Schwenklagen ein Steuersignal für eine Schlaufenlängenkorrektur durch den Antrieb 33 abgibt.

## Patentansprüche

1. Verfahren zur aseptischen Abfüllung von Nahrungs- oder Genußmitteln in Becher aus Kunststoffolie, bei dem in steriler Umgebung die Becherfolie (3) zur Becherausformung plastifiziert und anschließend verformt wird und die Becherausnehmungen (9') befüllt und mittels Deckelfolie (17) verschlossen werden, wobei die Becherfolie (3) vor dem Abfüllvorgang jeweils in einem Längenabschnitt zu Sterilisationszwecken mittels Dampf beaufschlagt und dabei an ihrer dem Dampfbeaufschlagungsbereich gegenüberliegenden Seite gekühlt wird, wobei die Deckelfolie (17) vor ihrer Zuführung zur Becherfolie (3) sterilisiert wird, und wobei die Deckel- und die Becherfolie nach ihrer Sterilisation und bis zu ihrer Vereinigung in keimfreier Umgebung geführt werden, **dadurch gekennzeichnet,** daß zu ihrer beidseitigen Sterilisation die Becherfolie in flachem Zustand vor ihrer Verformung in zwei aufeinanderfolgenden Behandlungsvorgängen im jeweils gleichen Längenabschnitt wechselseitig mit Dampf beaufschlagt und dabei gekühlt wird.

2. Verfahren zur aseptischen Abfüllung von Nahrungs- oder Genußmitteln in Becher aus Kunststoffolie, bei dem in steriler Umgebung die Becherfolie (3) zur Becherausformung plastifiziert und anschließend verformt wird und die Becherausnehmungen (9') befüllt und mittels Deckelfolie (17) verschlossen werden, wobei die Becherfolie (3) vor dem Abfüllvorgang sterilisiert und zur Sterilisation der Deckelfolie (17) diese vor ihrer Zuführung zur Becherfolie (3) jeweils in einem Längenabschnitt beidseitig mit Dampf beaufschlagt wird, und wobei die Deckel- und die Becherfolie nach ihrer Sterilisation und bis zu ihrer Vereinigung in keimfreier Umgebung geführt werden, **dadurch gekennzeichnet,** daß zur beidseitigen Sterilisation der aus Kunststoff bestehenden Deckelfolie (17) diese vor ihrer Zuführung zur Becherfolie (3) in zwei aufeinanderfolgenden Behandlungsvorgängen im jeweils gleichen Längenabschnitt wechselseitig mit Dampf beaufschlagt und dabei an ihrer dem Dampfbeaufschlagungsbereich gegenüberliegenden Seite gekühlt wird.

3. Verfahren nach Anspruch 1, **dadurch gekennzeichnet,** daß die Dampfbehandlung der Becherfolie (3) vor deren Plastifizierung vorgenommen wird.

4. Verfahren nach Anspruch 1 oder 2, **dadurch gekennzeichnet,** daß die Becherfolie (3) bzw. die Deckelfolie (17) im Dampfbeaufschlagungsbereich oberflächig auf ca. 120 - 145°C erwärmt wird und die Temperatur an der gekühlten Oberfläche der Folie auf etwa 60°C begrenzt wird.

5. Verfahren nach einem der Ansprüche 1 bis 4**, dadurch gekennzeichnet,** daß die Haltezeit für die Dampfbeaufschlagung etwa 5 bis 10 Sekunden beträgt.

6. Maschine zur aseptischen Abfüllung von Nahrungs- oder Genußmitteln mit einer Becherformvorrichtung (15), einer Abfüllvorrichtung (6), einer Versiegelungsvorrichtung (20), einer Sterilisationsvorrichtung (12, 19) für die Becher- und für die Deckelfolie und mit Fördermitteln zur taktweisen Bewegung von Becher- und Deckelfolie, **dadurch gekennzeichnet,** daß sie als Sterilisationsvorrichtung (12,19) für die Becherfolie (3) und/oder für die Deckelfolie (17) zwei hintereinander angeordnete Gruppen von plattenförmigen Werkzeugteilen umfaßt, die je einen Längenabschnitt der flachen Becher- bzw. Deckelfolie (3,17) zwischen sich aufnehmen, wobei ein Werkzeugteil als Kontaktkühlplatte (23,24) und der gegenüberliegende Werkzeugteil als Dampfverteilerplatte (25,26) ausgebildet ist und in der ersten Gruppe von Werkzeugteilen die Dampfverteilerplatte (25,26) der einen Folienseite und in der zweiten Gruppe der anderen Folienseite zugeordnet ist.

7. Maschine nach Anspruch 6, **dadurch gekennzeichnet,** daß die Dampfverteilerplatte (25,26) über ihre der Folie (3,17) zugewandte Fläche verteilt angeordnete Austrittsöffnungen (28) für den Dampf aufweist.

8. Maschine nach Anspruch 7, **dadurch gekennzeichnet,** daß die Austrittsöffnungen (28) als Düsen ausgebildet sind.

9. Maschine nach einem der Ansprüche 6 bis 8, **dadurch gekennzeichnet,** daß die beiden Werkzeuggruppen (23,25,24, 26) eine zur Umgebung hin abgedichtete, gekapselte Sterilisations-Einheit (12) bilden, die außerhalb eines mit Sterilluft beschickten Steriltunnels (13) einer Abfüllmaschine (1) diesem unmittelbar in Laufrichtung der zu sterilisierenden Folie (3,17) vorgeordnet ist.

10. Maschine nach einem der Ansprüche 6 bis 9, **dadurch gekennzeichnet,** daß zur Bewegung der Folie (3,17) durch die Sterilisationsvorrichtung (12,19,19') eine gesonderte Vorschubvorrichtung (33) vorgesehen ist, mittels der der Folie (3,17) bis zum Verlassen der Sterilisationsvorrichtung (12,19,19') eine von der Vorschubbewegung durch eine Hauptvorschubvorrichtung (38) in nachfolgenden Bereichen der Abfüllmaschine (1) unabhängige Bewegung erteilbar ist.

11. Maschine nach Anspruch 10, **dadurch gekennzeichnet,** daß die Vorschubvorrichtung (33) für die Sterilisationsvorrichtung(en) (12,19,19') der Becher- bzw. Deckelfolie (3,17) Vorschubbewegungen in einer Taktfolge und mit einer Weglänge erteilt, die einem Mehrfachen der Taktfolge und der Weglänge der Vorschubbewegung entspricht, die die Hauptvorschubvorrichtung (38) der Becherbahn (3) in den nachfolgenden Bereichen der Abfüllmaschine erteilt.

12. Maschine nach Anspruch 10 und 11, **dadurch gekennzeichnet,** daß der Steriltunnel (13) im Bereich hinter der Sterilisationsvorrichtung (12,19,19') einen erweiterten Aufnahmeraum (35) für eine Vorratsschlaufe (36) der Becher- bzw. Deckelfolie (3,17) aufweist.

13. Maschine nach einem der Ansprüche 6 bis 12, **dadurch gekennzeichnet,** daß das jeweils obere Plattenteil (23,26) jeder Werkzeuggruppe (23,25 bzw. 24,26) der Sterilisationsvorrichtung (12,19,19') ortsfest abgestützt und das jeweils untere Plattenteil (24,25) mittels eines Hubantriebs auf- und abbewegbar sowie mittels einer Verriegelungsvorrichtung in seiner oberen Betriebsstellung arretierbar ist.

14. Maschine nach einem der Ansprüche 6 bis 13, **dadurch gekennzeichnet,** daß jede Gruppe von Werkzeugteilen (23, 24,25,26) Platten einer zumindest der Bahnbreite der Folien (3,17) entsprechenden Arbeitsbreite und einer Arbeitslänge aufweist, die im wesentlichen der Weglänge der durch die Vorschubvorrichtung (33) der Folie (3,17) erteilten Vorschubbewegung entspricht.

## Claims

1. A method for the aseptic filling of foodstuffs or other edible products into tubs consisting of synthetic plastics sheet and in which, for shaping the tubs, the tub sheet (3) is plasticised and then shaped in a sterile environment and the tub recesses (9') are filled and sealed by a top foil (17), whereby, prior to the filling process, the tub sheet (3) is for sterilisation purposes exposed to steam over a portion of its length while on its side opposite the area which is exposed to steam, the tub sheet is cooled, the top foil (17) being sterilised prior to being fed to the tub sheet (3), and whereby, following their sterilisation and until they are combined, the top foil and tub sheet are conveyed in a sterile environment, characterised in that for its bilateral sterilisation, the tub sheet, while in its flat state and prior to being shaped, is alternately exposed to steam in two successive treatment processes affecting in each case the same portion of length, being cooled at the same time.

2. A method for the aseptic filling of foodstuffs or other edible products into tubs consisting of synthetic plastics sheet in which, for shaping the tubs, the tub sheet (3) is plasticised and then shaped in a sterile environment and the tub recesses (9') are filled and sealed by a top foil (17), whereby prior to the filling process the tub sheet (3) is sterilised and for sterilisation of the top foil (17), this latter, prior to being fed to the tub sheet (3), is exposed to steam on both sides over one length portion, and whereby, following their sterilisation and until they are combined, the top foil and tub sheet are conveyed in a sterile environment, characterised in that for the top foil (17) which consists of a synthetic plastics material to be sterilised on both sides, and prior to its being fed to the tub sheet (3), the top foil (17) is, in two successive treatment processes, alternately exposed to steam over in each case the same length portion, being cooled thereby on its side opposite the area which is exposed to the steam.

3. A method according to Claim 1, characterised in that the steam treatment of the tub sheet (3) takes place prior to its plasticisation.

4. A method according to Claim 1 or 2, characterised in that the tub sheet (3) or top foil (17) has its area which is exposed to steam heated on its surface to approx. 120 to 145°C, the temperature at the cooled surface of the sheet being limited to about 60°C.

5. A method according to one of Claims 1 to 4, characterised in that the time for which the exposure to steam is maintained amounts to about 5 to 10 seconds.

6. A machine for the aseptic filling of foodstuffs or other edible products, with a tub-shaping apparatus (15), a filling apparatus (6), a sealing apparatus (20), a sterilisation apparatus (12, 19) for the tub sheet and for the top foil and with conveying means for the cyclic movement of tub sheet and top foil, characterised in that the machine comprises as a sterilising apparatus (12, 19) for the tub sheet (3) and/or for the top foil (17) two serially disposed groups of plate-like tool parts which accommodate between them one length portion at a time of the flat tub sheet or top foil (3, 17), one tool part being constructed as a contact cooling plate (23, 24) while the opposite tool part is constructed as a steam-distributing plate (25, 26) and in the first group of tool parts the steam-distributor plate (25, 26) is associated with one side of the sheet and with the other side of the sheet in the second group.

7. A machine according to Claim 6, characterised in that the steam-distributor plate (25, 26) has, for the steam, outlet orifices (28) distributed over its surface which faces the sheet (3, 17).

8. A machine according to Claim 7, characterised in that the outlet orifices (28) are constructed as jets.

9. A machine according to one of Claims 6 to 8, characterised in that the two tool groups (23, 25, 24, 26) form one enclosed sterilising unit (12) sealed in respect of the ambient and situated outside and upstream of a sterile tunnel (13) filled with sterile air and part of a filling machine (1), being disposed directly upstream of the said tunnel in the direction of movement of the sheet (3, 17) which is to be sterilised.

10. A machine according to one of Claims 6 to 9, characterised in that for moving the sheet (3, 17) through the sterilising apparatus (12, 19, 19'), a separate feed device (33) is provided, by means of which, until it leaves the sterilising apparatus (12, 19, 19'), a movement independent of the feed movement through a main feed apparatus (38) can be imparted to the sheet in subsequent parts of the filling machine (1).

11. A machine according to Claim 10, characterised in that the feed apparatus (33) for the sterilising apparatus(es) (12, 19, 19') imparts to the tub or top foil (3, 17) feed movements in a timed sequence and with a path length which corresponds to a multiple of the timed sequence and path length of the feed device which the main feed apparatus (38) imparts to the tub sheet (3) in the subsequent areas of the filling machine.

12. A machine according to Claim 10 and 11, characterised in that the sterile tunnel (13) has in the area following the sterilising apparatus (12, 19, 19') a widened out receiving space (35) for a supply loop (36) of tub and top sheet (3, 17).

13. A machine according to one of Claims 6 to 12, characterised in that whichever is the upper plate part (23, 26) of each tool group (23, 25 or 24, 26) of the sterilising apparatus (12, 19, 19') is supported in stationary manner while whichever is the bottom plate part (24, 25) can be moved upwards and downwards by means of a lifting drive and locked in its upper operating position by means of a locking device.

14. A machine according to one of Claims 6 to 13, characterised in that each group of tool parts (23, 24, 25, 26) comprises plates having a working width and a working length corresponding at least to the width of the sheets (3, 17) and which correspond substantially to the path length of the feed movement imparted to the sheet (3, 17) by the feed apparatus (33).

## Revendications

1. Procédé pour le remplissage aseptique de dentées alimentaires ou de friandises dans une boîte faite avec une feuille de plastique dans le cas duquel on plastifie dans l'environnement stérile la feuille pour la boîte (3) pour la démouler et ensuite on la déforme et on remplit les éléments de la boîte (9') et on les ferme au moyen de la feuille de couverture (17), la feuille pour la boîte (3) étant soumise à un jet de vapeur avant le processus de remplissage respectivement dans une section longitudinale aux fins de stérilisation et ce faisant étant refroidie sur sa face opposée à la zone exposée à la vapeur, la feuille de couverture (17) étant stérilisée avant d'être amenée à la feuille pour la boîte (3), les feuilles pour la boîte et la couverture étant introduites après leur stérilisation et jusqu'à leur réunion dans une atmosphère exempte de germes,
caractérisé en ce que
pour être stérilisée des deux côtés, la feuille pour la boîte est exposée à la vapeur dans une position à plat avant sa déformation en deux phases de traitement consécutives dans respectivement la même section longitudinale alternativement d'un côté et de l'autre et est ce faisant refroidie.

2. Procédé pour le remplissage aseptique de denrées alimentaires ou de friandises dans une boîte réalisée à partir d'une feuille en matière plastique, dans le cas duquel la feuille pour la boîte (3) est plastifiée dans une atmosphère stérile pour le démoulage de la boîte et est ensuite déformée et les évidements (9') de la boîte sont remplis et sont fermés au moyen de la feuille de couverture (17), la feuille pour la boîte (3) étant stérilisée avant le processus de remplissage et étant soumise pour la stérilisation de la feuille de couverture (17) avant d'être mise sur la feuille pour la boîte (3) respectivement dans une même section longitudinale des deux côtés à la vapeur et la feuille de couverture et la feuille pour la boîte après leur stérilisation et jusqu'à leur réunion étant mises dans une atmosphère exempte de germes,
caractérisé en ce que
pour la stérilisation des deux côtés de la feuille de couverture (17) réalisée en matière plastique, on la soumet, avant de l'amener à la feuille pour la boîte (3), à deux étapes consécutives de traitement sur des sections ayant respectivement la même longueur alternativement d'un côté et de l'autre à un jet de vapeur et ce faisant est refroidie sur sa face opposée à la zone exposée à la vapeur.

3. Procédé selon la revendication 1,
caractérisé en ce que
le traitement à la vapeur de la feuille pour la boîte (3) a lieu avant sa plastification.

4. Procédé selon la revendication 1 ou 2,
caractérisé en ce que
la feuille pour la boîte (3) ou la feuille de couverture (17) est chauffée dans la zone exposée à la vapeur superficiellement à environ 120 à 145°C et la température sur la face refroidie de la feuille est limitée à environ 60°C.

5. Procédé selon l'une des revendications 1 à 4,
caractérisé en ce que
le temps de maintien pour l'exposition à la vapeur atteint environ 5 à 10 secondes.

6. Machine pour le remplissage aseptique de denrées alimentaires ou de friandises, avec un dispositif de formage d'une boîte (15), un dispositif de remplissage (6), un dispositif de scellement (20), un dispositif de stérilisation (12, 19) pour la feuille pour la boîte et pour la feuille de couverture et avec des moyens de transport pour faire avancer de façon cadencée la feuille pour la boîte et la feuille de couverture,
caractérisée en ce que
elle comprend comme dispositif de stérilisation (12, 19) de la feuille pour la boîte (3) et/ou de la feuille de couverture (17) deux groupes, disposés l'un derrière l'autre de parties d'outils en forme de plaques, qui reçoivent respectivement chacun une section longitudinales de la feuille plate pour la boîte ou de couverture (3, 17) entre elles, une partie d'outil étant constituée comme plaque de refroidissement par contact (23, 24) et la partie d'outil située en regard étant constituée sous la forme d'une plaque de répartition de la vapeur (25, 26) et dans le premier groupe des parties d'outil, la plaque de répartition de la vapeur (25, 26) est associée à l'une des faces de la feuille et dans le second groupe elle est associée à l'autre face de la feuille.

7. Machine selon la revendication 6,
caractérisée en ce que
la plaque de répartition de la vapeur (25, 26) présente des orifices de sortie (28) disposés de façon répartie sur sa face tournée vers la feuille (3, 17) pour la vapeur.

8. Machine selon la revendication 7,
caractérisée en ce que
les orifices de sortie (28) sont constitués sous la forme de buses.

9. Machine selon l'une des revendications 6 à 8,
caractérisée en ce que
les deux groupes d'outils (23, 25 ; 24, 26) forment une unité de stérilisation (12) encapsulée, rendue étanche par rapport à l'environnement qui, en dehors d'un tunnel stérile (13), garni d'air stérile, est disposée directement en avant d'une machine de remplissage (1) dans le sens de circulation de la feuille (3, 17) à stériliser.

10. Machine selon l'une des revendications 6 à 9,
caractérisée en ce que
pour déplacer la feuille (3, 17) à travers le dispositif de stérilisation (12, 19, 19') on prévoit un dispositif d'avancement particulier (33) au moyen duquel on peut imprimer à la feuille (3, 17) un mouvement indépendant du mouvement d'avancement par un dispositif d'avancement principal (38) dans des zones suivantes de la machine de remplissage (1) jusqu'à ce qu'elle ait quitté le dispositif de stérilisation (12, 19, 19').

11. Machine selon la revendication 10,
caractérisée en ce que
le dispositif d'avancement (33) pour le(s) dispositifs(s) de stérilisation (12, 19, 19') imprime à la feuille (3, 17) pour la boîte ou pour la couverture des mouvements d'avancement à une cadence et avec une longueur d'avancement, qui correspond à un multiple de la cadence et de la longueur du mouvement d'avancement que le dispositif principal d'avancement (38) imprime à la bande (3) pour la boîte dans les zones suivantes de la machines de remplissage.

12. Machine selon les revendications 10 et 11,
caractérisée en ce que
le tunnel stérile (13) présente dans la zone derrière le dispositif de stérilisation (12, 19, 19') un espace de réception élargi (35) pour une boucle d'alimentation (36) de la feuille pour la boîte ou de la feuille de couverture (3, 17).

13. Machine selon l'une des revendications 6 à 12,
caractérisée en ce que
la partie respectivement supérieure de la plaque (23, 26) de chaque groupe d'outil (23, 25 ou 24, 26) prend appui de façon fixe sur le dispositif de stérilisation (12, 19, 19') et la partie respectivement inférieure de la plaque (24, 25) peut être soulevée ou abaissée au moyen d'un mécanisme de levage et peut être bloquée au moyen d'un dispositif de verrouillage dans sa position supérieure de fonctionnement.

14. Machine selon l'une des revendications 6 à 13,
caractérisée en ce que
chaque groupe de parties d'outils (23, 24, 25, 26) présente des plaques qui ont une largeur de travail correspondant au moins à la largeur de bande des feuilles (3, 17) et une longueur de travail, qui correspond sensiblement à la longueur du mouvement d'avancement imprimé par le dispositif d'avancement (33) de la feuille (3, 17).
